# DEMANDE DE BREVET EUROPEEN

(11) **EP 1 405 630 A1**
(43) Date de publication de la demande: **07.04.2004**
(21) Numéro de dépôt: 03292104.1
(22) Date de dépôt: 27.08.2003
(51) Int. Cl.: A61K 7/20

(54) **Procédé d'obtention de dégagements gazeux controlés à partir d'un mélance de composés chimiques et applications de ce procédé notamment à la réalisation d'une pate dentifrice**

(30) Priorité: 03.09.2002 FR 0210947
(71) Demandeur: Cartier, Maurice, 94440 Marolles en Brie (FR); Liance, Loic, 75017 Paris (FR)
(72) Inventeur: Cartier, Maurice, 94440 Marolles en Brie (FR); Liance, Loic, 75017 Paris (FR)
(74) Mandataire: Keib, Gérard

(57) **Abrégé**

Procédé d'obtention de dégagements gazeux contrôlés à partir d'un mélange de composés chimiques contenus dans un récipient unique, ce mélange comprenant :
- un composé chimique apte à générer le dégagement d'un premier gaz, lorsqu'il est mis en contact avec un liquide activateur,
- au moins un second composé apte à générer le dégagement d'un second gaz différent du premier, lorsqu'il est mis en contact avec ledit liquide, le premier composé chimique et le second étant incompatibles entre eux,
- le procédé est caractérisé par la mise en oeuvre de moyens d'isolation physique ou chimique et,
- par la mise en contact des deux composés contenus dans le récipient unique avec le liquide activateur de façon à provoquer le dégagement simultané du premier et du second gaz.

## Description

La présente invention concerne un procédé d'obtention de dégagements gazeux contrôlés à partir d'un mélange de composés chimiques.

L'invention vise également l'application du procédé précité à la réalisation d'un produit cosmétique apte à provoquer, lorsqu'il est mis en présence d'eau, un dégagement simultané de gaz carbonique et d'oxygène naissant.

Selon une forme de réalisation préférée de l'invention, le produit cosmétique précité est une pâte dentifrice.

On sait que l'oxygène naissant, c'est-à-dire l'oxygène atomique, a des propriétés oxydantes très puissantes qui le rendent capable de décomposer des substances organiques.

L'un des buts de la présente invention est d'utiliser cette propriété pour créer un produit cosmétique et en particulier une pâte dentifrice dans laquelle le dégagement d'oxygène naissant obtenu lors du brossage des dents est capable d'éliminer efficacement la plaque dentaire et les détritus organiques logés dans les espaces interdentaires.

Les tentatives effectuées jusqu'à présent, telles que décrites notamment dans le brevet français n° 98.16329 du 23 décembre 1998 au nom de la Société CLEANI, n'ont pas permis d'exploiter d'une manière optimale les propriétés oxydantes de l'oxygène naissant. En effet, ces tentatives ont montré que le dégagement d'oxygène naissant seul n'était pas suffisant pour obtenir un résultat exploitable.

Le but de la présente invention est de créer un procédé permettant d'obtenir un dégagement d'oxygène naissant optimal, ce procédé étant également applicable à l'obtention d'autres dégagements gazeux.

L'invention vise ainsi un procédé d'obtention de dégagements gazeux contrôlés à partir d'un mélange de composés chimiques contenus dans un récipient unique, ce mélange comprenant :
- un composé chimique apte à générer le dégagement d'un premier gaz, lorsqu'il est mis en contact avec un liquide activateur,
- au moins un second composé apte à générer le dégagement d'un second gaz différent du premier, lorsqu'il est mis en contact avec ledit liquide, le premier composé chimique et le second étant incompatibles entre eux,
- le procédé mettant en oeuvre des moyens d'isolation physique ou chimique et, la mise en contact des deux composés contenus dans le récipient unique avec le liquide activateur de façon à provoquer le dégagement simultané du premier et du second gaz, le procédé étant caractérisé en ce que le liquide activateur est l'eau, en ce que le premier gaz est du gaz carbonique, le composé chimique apte à générer du gaz carbonique est un acide carboxylique réagissant avec un carbonate.

Les essais ont montré que la simultanéité favorisait le dégagement de l'un par rapport à l'autre gaz.

Dans une application préférée de l'invention le second gaz est de l'oxygène naissant.

Dans cette application préférée de l'invention, le dégagement d'oxygène naissant est favorisé par le dégagement simultané du gaz carbonique.

Le mérite de la présente invention réside dans le fait d'avoir obtenu ces dégagements gazeux simultanés à partir de composés chimiques incompatibles entre eux, contenus dans un récipient unique, en les isolant physiquement ou chimiquement, tant qu'ils ne sont pas mis en contact avec le liquide, en particulier l'eau qui déclenche les réactions chimiques générant les dégagements gazeux.

L'acide carboxylique réagissant avec un carbonate pour libérer le gaz carbonique, peut être par exemple de l'acide fumarique, tartrique, citrique, ascorbique ou autres. Ces acides sont parfaitement adaptés à l'application préférée de l'invention, à savoir la pâte dentifrice.

Dans cette application préférée, le composé chimique apte à générer de l'oxygène naissant est un persel tel qu'un peroxyde, un persulfate, perchlorate, un perborate, ou un percarbamate.

Le persel préféré est le perborate de sodium, en raison de sa faible toxicité et de sa vitesse de décomposition.

Le perborate de sodium se décompose en présence de l'acide carboxylique pour produire de l'oxygène naissant.

Dans une version préférée de l'invention, lesdits moyens pour isoler physiquement les deux composés chimiques sont constitués par un excipient d'enrobage apte à isoler les deux composés chimiques, tel que de l'huile de paraffine.

Dans l'application préférée de l'invention c'est-à-dire la pâte dentifrice, l'excipient doit également être conforme aux législations en vigueur pour les produits alimentaires, médicamenteux et cosmétiques.

Dans cette application, à titre d'exemple, l'excipient est constitué par le mélange des constituants suivants : triéthanolamine, lauryl sulfate de sodium et huile de paraffine ou autres ayant les mêmes caractéristiques physicochimiques.

Dans une autre version de l'invention, lesdits moyens pour isoler chimiquement les deux composés chimiques consistent à maintenir le pH du milieu contenant les deux composés à une valeur supérieure à 7 et à mettre en oeuvre un catalyseur lors de la mise en contact avec de l'eau pour amener le pH à une valeur inférieure à 7 afin de déclencher les réactions engendrant les dégagements gazeux.

A titre d'exemple, la composition d'une pâte dentifrice selon l'invention peut être la suivante :
Les pourcentages de triéthanolamine sont en accord avec le J.O. du 23 février 2001, c'est-à-dire 2,5%.
Il en a été de même pour le fluorure de sodium (0,15%) et l'oxygène actif.
Les variations de composants de l'excipient évoluent suivant la viscosité recherchée et l'emploi auquel les mélanges sont destinés.
D'autre part, il est possible d'employer deux types de carbonate de calcium (lourd et léger) et d'huile de paraffine soit épaisse soit légère.
Le tableau suivant résume les différentes possibilités en pourcentage pour l'exemple d'une pâte dentifrice :
   - triéthanolamine : 2,5 gr
   - perborate de sodium : 0,25 gr
   - acide tartrique : 2 gr
   - huile de paraffine (épaisse ou légère) : 5 à 35 gr
   - glycérine : 10 à 60 gr
   - carbonate de calcium (lourd ou léger) : 15 à 40 gr
   - bicarbonate de sodium : 2 gr
   - fluorure de sodium : 0,15 gr
   - butyl paraben: 0,001 gr
   - néohespéridine )
   - colorant ) q.s. ad libitum
   - essence de menthe )

Ces composés peuvent être contenus dans un tube classique pour pâte dentifrice.

Le dégagement simultané de gaz carbonique et d'oxygène naissant est provoqué lors de la mise en contact avec l'eau, de la pâte dentifrice appliquée sur une brosse à dents. Ce dégagement gazeux se produit lorsque la pâte dentifrice est humidifiée et appliquée sur les dents.

Les essais ont montré que le dégagement d'oxygène naissant conjointement avec celui du gaz carbonique était favorisé par leur simultanéité. Il en résulte une augmentation de l'action de l'oxygène naissant sur la plaque dentaire, les résidus alimentaires situés dans les espaces interdentaires et un blanchiment accentué des dents.

Bien entendu, l'invention n'est pas limitée à l'application en tant que pâte dentifrice que l'on vient de décrire.

Ainsi le procédé selon l'invention est utilisable dans toutes les applications cosmétiques, thérapeutiques et autres, dans lesquelles il est souhaitable d'obtenir le dégagement simultané de plusieurs gaz à partir d'un mélange de plusieurs composés chimiques incompatibles entre eux, contenus dans un récipient unique.

En effet, la découverte que le dégagement simultané de plusieurs gaz favorise le dégagement de l'un d'eux ayant des propriétés spécifiques rend l'invention utilisable pour une application dans laquelle ces propriétés spécifiques sont particulièrement recherchées.

## Revendications

1. Procédé d'obtention de dégagements gazeux contrôlés à partir d'un mélange de composés chimiques contenus dans un récipient unique, ce mélange comprenant :
- un composé chimique apte à générer le dégagement d'un premier gaz, lorsqu'il est mis en contact avec un liquide activateur,
- au moins un second composé apte à générer le dégagement d'un second gaz différent du premier, lorsqu'il est mis en contact avec ledit liquide, le premier composé chimique et le second étant incompatibles entre eux,
- le procédé mettant en oeuvre des moyens d'isolation physique ou chimique et, la mise en contact des deux composés contenus dans le récipient unique avec le liquide activateur de façon à provoquer le dégagement simultané du premier et du second gaz, le procédé étant **caractérisé en ce que** le liquide activateur est l'eau, **en ce que** le premier gaz est du gaz carbonique, le composé chimique apte à générer du gaz carbonique est un acide carboxylique réagissant avec un carbonate.

2. Procédé selon la revendication 1, **caractérisé en ce que** le second gaz est de l'oxygène naissant.

3. Procédé selon la revendication 2, **caractérise en ce que** le composé chimique apte à générer de l'oxygène naissant est un persel tel qu'un peroxyde, un persulfate, perchlorate, un perborate ou un carbamate.

4. Procédé selon l'une des revendications 1 à 3, **caractérisé en ce que** lesdits moyens pour isoler physiquement les deux composés chimiques incompatibles entre eux sont constitués par un excipient d'enrobage apte à isoler les deux composés chimiques.

5. Procédé selon la revendication 4, **caractérisé en ce que** ledit excipient est constitué par le mélange des constituants suivants, ou autres ayant les mêmes caractéristiques physicochimiques :
- triéthanolamine
- lauryl sulfate de sodium
- huile de paraffine.

6. Procédé selon l'une des revendications 1 à 3, **caractérisé en ce que** lesdits moyens pour isoler chimiquement les deux composés consistent à maintenir le pH du milieu à une valeur supérieure à 7 et à mettre en oeuvre un catalyseur lors de la mise en contact avec de l'eau pour amener le pH à une valeur inférieure à 7 afin de déclencher les réactions engendrant les dégagements gazeux.

7. Application du procédé selon l'une des revendications 3 à 6, à la réalisation d'un produit cosmétique apte à provoquer lorsqu'il est mis en présence d'eau un dégagement simultané de gaz carbonique et d'oxygène naissant.

8. Application selon la revendication 7, **caractérisée en ce que** ledit produit cosmétique est une pâte dentifrice dans laquelle le dégagement simultané de gaz carbonique et d'oxygène naissant est provoqué lors de la mise en contact de la pâte dentifrice avec l'eau (appliquée sur une brosse à dents) le dégagement gazeux se prolongeant lorsque la pâte dentifrice est appliquée sur les dents.

9. Pâte dentifrice utilisée dans l'application selon la revendication 8, **caractérisée en ce qu'**elle renferme les composés suivants :
- triéthanolamine
- perborate de sodium
- acide tartrique
- glycérine
- huile de paraffine
- bicarbonate de sodium
- carbonate de calcium
- lauryl sulfate de sodium
- fluorure de sodium
- produits colorant, aromatisant et sucrant.
